# EUROPEAN PATENT APPLICATION

(11) **EP 2 380 886 A1**
(43) Date of publication of application: **26.10.2011**
(21) Application number: 10160864.4
(22) Date of filing: 23.04.2010
(51) Int. Cl.: C07D 279/08

(54) **Process for the preparation of 2-aminosubstituted 1,3-benzothiazine-4-ones**

(71) Applicant: Makarov Vadim A., Moskow 129090 (RU)
(72) Inventor: Makarov Vadim A., Moskow 129090 (RU)
(74) Representative: HOFFMANN EITLE

(57) **Abstract**

A process for preparing 2-aminosubstituted 1,3-benzothiazine-4-ones is provided which comprises the following step:

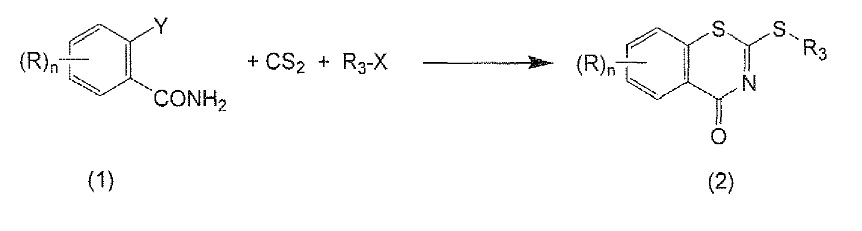

wherein Y is halogen;
R is independently selected from C₁₋₆-alkyl which may optionally be substituted by halogen, -NO₂, halogen, -CHO -COOR₄ (wherein R₄ is hydrogen or C₁₋₆-alkyl) and -CN;
R₃ is C₁₋₃-alkyl which may be substituted by halogen;
X is halogen; and
n is 0 or an integer of 1 to 4; if n is 2, 3 or 4, multiple Rs may be the same or different.

## Description

### Technical Field

The present invention relates to a novel process for the preparation of 2-aminosubstituted 1,3-benzothiazine-4-ones, and to intermediates for use in this process.

### Background

2-Aminosubstituted 1,3-benzothiazine-4-ones can be used as drugs for the treatment of mammals diseases, for example, infectious diseases of mammals (humans and animals) caused by bacteria, especially diseases like tuberculosis (TB) and leprosy caused by mycobacteria.

Specific 2-aminosubstituted 1,3-benzothiazine-4-ones are disclosed e.g. in WO 2007/134625 and WO 2009/010163.

Several methods for synthesis of 2-aminosubstituted 1,3-benzothiazine-4-ones are described in the prior art. These methods include:
1) the reaction of thiocyanate salts with 2-chlorobenzylchloroanhydride, and subsequent treatment of the reaction mass with the corresponding amine (see, for example, Coll. Czech. Chem. Commun., 1982, 47, 3268-3282; Coll. Czech. Chem. Commun., 1983, 48, 3315-3328; Coll. Czech. Chem. Commun., 1983, 48, 3427-3432);
2) the condensation reaction of 3,4-disubstituted-6-mercaptobenzoic acids with a suitable cyanamide (see US 3,522,247);
3) the conversion of a 2-halogen-4H-1,3-benzothiazin-4-one with an appropriate amine (see US 3,470,168).

None of these methods allows for preparing 2-aminosubstituted 1,3-benzothiazine-4-ones in an acceptable yield. In addition, the key intermediate of method 3, i.e. the 2-halogen-1,3-4H-benzothiazone, is not readily accessible.

Recent methods for preparing 2-aminosubstituted 1,3-benzothiazine-4-ones are described in WO 2007/134625 and WO 2009/010163 which disclose the following processes:

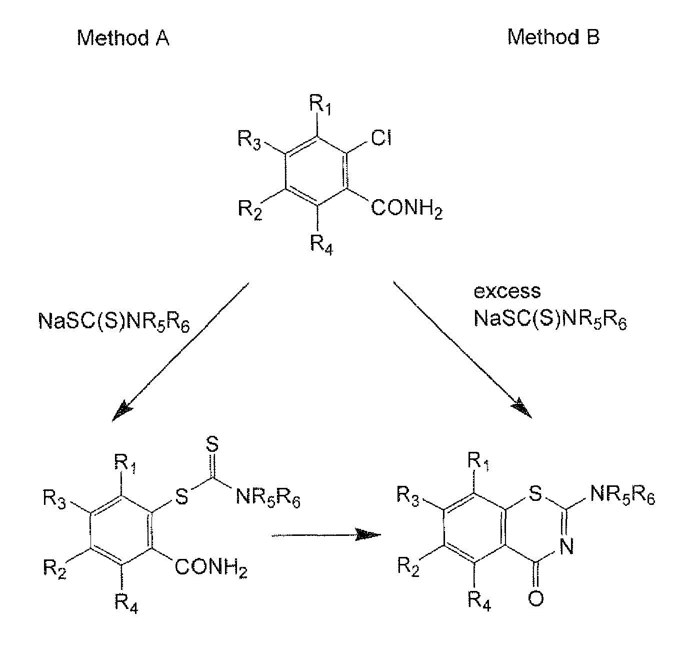

However, these processes result in the formation of free hydrogen sulfide which can react with the starting materials and the final product, thereby giving rise to several byproducts.

An alternative process taught in WO 2009/01063 is Method C:

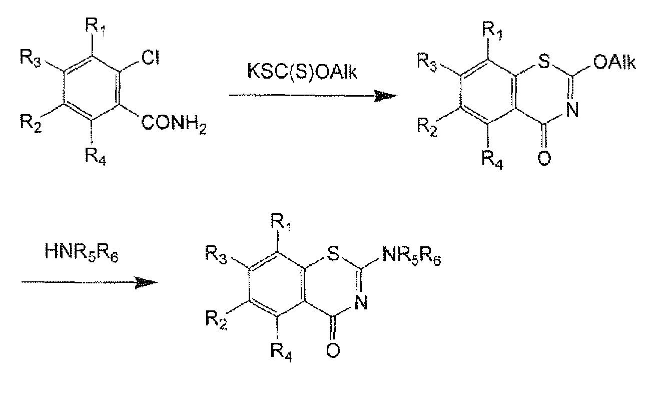

However, the yield of this method is unsatisfactory, in particular for secondary amines.

In view of this background, it is highly desirable to provide a process for preparing 2-aminosubstituted 1,3-benzothiazine-4-ones which results in a high yield and purity, and can easily be carried out.

### Summary of the Invention

The above object is surprisingly solved by a process for preparing 2-aminosubstituted 1,3-benzothiazine-4-ones which comprises the following step: 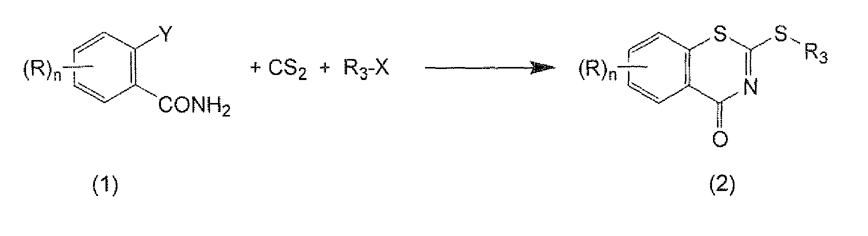 wherein
Y is halogen;
R is independently selected from C₁₋₆-alkyl which may optionally be substituted by halogen, -NO₂, halogen, -CHO, -COOR₄ (wherein R₄ is hydrogen or C₁₋₆-alkyl) and -CN;
R₃ is C₁₋₃-alkyl which may be substituted by halogen;
X is halogen; and
n is 0 or an integer of 1 to 4, and if n is 2, 3 or 4, multiple Rs may be the same or different.

### Detailed Description of the Invention

The present invention provides a process for preparing 2-aminosubstituted 1,3-benzothiazine-4-ones which comprises the following step: 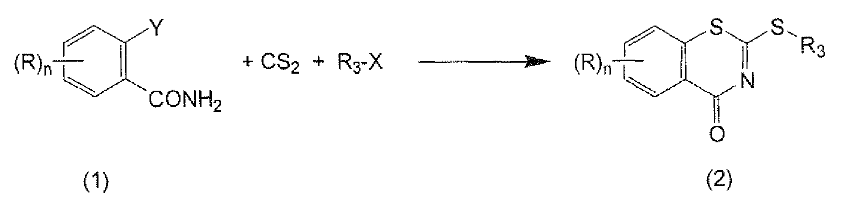 wherein
Y is halogen, preferably chlorine;
R is independently selected from C₁₋₆-alkyl which may optionally be substituted by halogen, -NO₂, halogen, -CHO, -COOR₄ (wherein R₄ is hydrogen or C₁₋₆-alkyl) and -CN;
R₃ is C₁₋₃-alkyl which may be substituted by halogen, preferably methyl;
X is halogen, preferably iodine; and
n is 0 or an integer of 1 to 4, and if n is 2, 3 or 4, multiple Rs may be the same or different.

In a preferred embodiment of the present invention, the process comprises the following step: 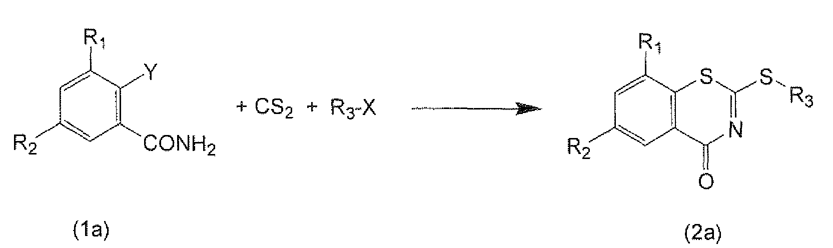 wherein
Y is halogen, preferably chlorine;
R₁ is H, -CF₃, -NO₂ or halogen;
R₂ is -COOR₄ (wherein R₄ is hydrogen or C₁₋₆-alkyl), -CN, halogen, or -CF₃;
R₃ is C₁₋₃-alkyl which may be substituted by halogen, preferably methyl; and
Y is halogen, preferably iodine.

The reaction is preferably carried out in the presence of a base, more preferably in the presence of an alkali metal hydroxide, most preferably in the presence of LiOH, NaOH or KOH. The alkali metal hydroxide is advantageously employed in a molar ratio of 1-4, particularly 2-2.5, based on the 2-halogenobenzamide derivative of formula (1).

Carbon disulfide (CS₂) is suitably employed in a molar ratio of 1-10, preferably 2-5, based on the 2-halogenobenzamide derivative of formula (1).

The alkylating agent R₃-X is preferably CH₃I or CH₂I₂. This alkylating agent is preferably employed in a molar ratio of 1-10, preferably 2-5, based on the 2-halogenobenzamide derivative of formula (1).

The reaction may be conducted in an appropriate solvent. Preferably, the reaction is conducted in DMSO.

When preparing 2-aminosubstituted 1,3-benzothiazine-4-ones, the 2-alkylthio-1,3-benzothiazine-4-one of formula (2) is further reacted with a suitable amine, such as ammonia, a primary or secondary amine.

The reaction according to the present invention can be used to prepare various 2-amino-1,3-benzothiazine-4-ones. Preferred embodiments of these 2-amino-1,3-benzothiazine-4-ones are disclosed in WO 2007/134625, WO 2009/010163 and applicant's copending application EP 09 003 876.1, the disclosure of these compounds is herewith incorporated by reference.

In an especially preferred embodiment of the invention, the reaction with the amine can be depicted by the following scheme: 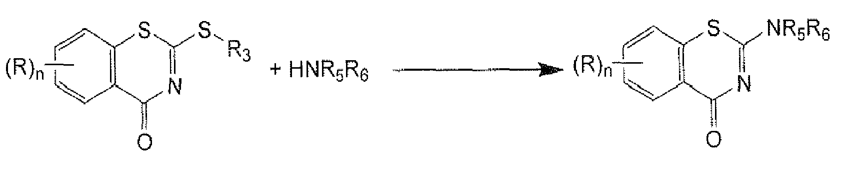 wherein R, R₃ and n are as defined above;
R₅ and R₆ are independently selected from H, a saturated or unsaturated, linear, branched or cyclic aliphatic radical having 1-8 carbon atoms, 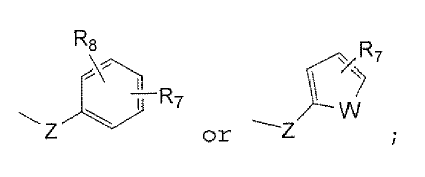 wherein
Z is saturated or unsaturated, linear or branched aliphatic radical having 1-5 carbon atoms;
W is O, NH or N-C₁₋₆-alkyl;
R₇ and R₈ are independently selected from H, C₁₋₆-alkyl, -OH, -OC₁₋₆-alkyl, halogen, or -CN;
R₅ and R₆ may together represent 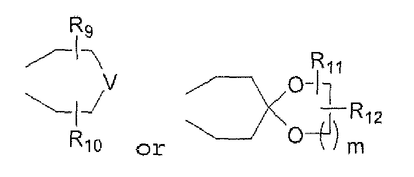 wherein V is O, S, C=O, CHOH, C(R₁₁)₂, NR₁₂;
R₉ and R₁₀ are H, C₁₋₃-alkyl;
R₁₁ is H, a saturated or unsaturated, linear, branched or cyclic aliphatic radical having 1-8 carbon atoms, -OH, -OC₁₋₆-alkyl, phenyl, benzyl, benzoyl, halogen; and multiple R₁₁s may be the same or different;
R₁₂ is methyl, benzyl, benzoyl or phenyl (which may be substituted by C₁₋₆-alkyl, -OH, -OC1-6-alkyl, halogen or -CN); and
m is an integer of 1-3;

In a further preferred embodiment of the invention, the reaction with the amine can be depicted by the following scheme: 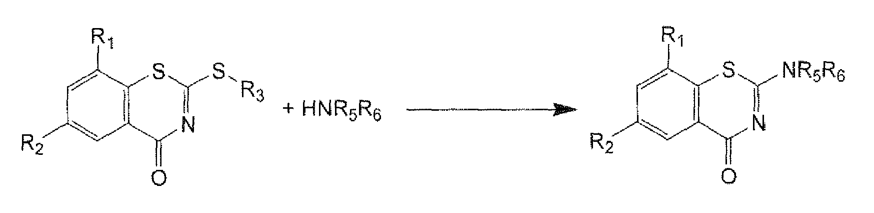 wherein R₁, R₂, R₃, R₅ and R₆ are as defined above.

The reaction with the amine may be conducted in an appropriate solvent, such as an alcoholic solvent (particularly methanol, ethanol and isopropanol), chloroform, tetrachlorometane, etc.

The amine is preferably used in a molar ratio of 1-2, preferably 1-1.2, based on the 2-alkylthio-1,3-benzothiazine-4-one of formula (2).

The 2-amino-1,3-benzothiazine-4-ones may be further processed into a suitable pharmaceutical composition, e.g. by compounding with appropriate excipients.

The present invention will hereinafter be described in more detail by way of the following non-limiting examples.

### Examples

Chemicals and solvents were purchased from Alfa-Aesar (GB) or from Aldrich Co. (Sigma-Aldrich Company, St-Louis, US). They were used without additional purification.

Melting points were determined according to the BP procedure and are uncorrected (Electrothermal 9001, GB).

If analyses are indicated only by the symbols of the elements, analytical results are within ±0.3% of the theoretical values (Carlo-Erba 5500, Italy).

NMR spectra were determined with a Varian Unity Plus 400 (USA). Shifts for ¹H NMR are reported in ppm downfield from TMS (δ).

Mass spectra were obtained using a Finnigan SSQ-700 (USA) instrument with direct injection.

Reactions and purity of compounds were controlled by TLC using Silicagel 60 F₂₅₄ aluminium sheets (Merck Co, Germany).

### Example 1

### 2-methylthio-8-nitro-6-trifluoromethyl-4H-1,3-benzothiazin-4-one (compound 1)

### Method A

Sodium hydroxide (0.9 g; powder) was dissolved in 10 ml DMSO, and 2.1 mL of carbon disulfide was added at a temperature of 10-15°C. 3.0 g of 2-chloro-3-nitro-5-trifluoromethylbenzamide was added to the solution in small portions at a temperature of 10°C. After 15 minutes, 0.7 mL of MeI was added at a temperature of 10-20°C. The reaction was allowed to proceed for 30 min, and subsequently 100 mL of water was added. The resulting yellow solid was separated by filtration.
Yield: 47%
mp: 200-203°C(ethyl acetate)
MS (m/z): 322 (M⁺)
¹H NMR (DMSO-d₆): δ 8.95 and 8.81 (two 1H, two s, 2CH), 2.73 (3H, s, CH₃) ppm
Anal. (C₁₀H₅F₃N₂O₃S₂):

| | |
|---|---|
| Calc.: | C, 37.28; H, 1.56; N, 8.69; S, 19.90 |
| Found: | C, 37.01; H, 1.53; N, 8.74; S, 20.11 |

### Method B

The reaction was conducted in a manner similar to method A, but the final product was crystallized from isopropanol.

### Method C

The reaction was conducted in a manner similar to method A, but the final product was crystallized from ethanol.

### Method D

Sodium hydroxide (0.3 g; powder) was added to a solution of 1.0 g of 2-chloro-3-nitro-5-trifluoromethylbenzamide in 10 ml DMSO at a temperature 10°C. 0.70 mL of carbon disulfide was added to the solution at a temperature of 10°C, and the reaction mixture allowed to stand for 15 minutes. 0.23 mL of MeI was added to the reaction mixture at a temperature of 10-20°C. The reaction mixture was allowed to stand for another 30 min, subsequently 100 mL of water was added. The resulting yellow solid was separated by filtration.
Yield: 39%
mp: 200-203°C (purified by crystallization according Methods A, B or C)

### Example 2

### 2-methylthio-6,8-bis(trifluoromethyl)-4H-1,3-benzothiazin-4-one (compound 2)

0.88 g of 2-chloro-3,5-ditrifluoromethylbenzamide was dissolved in 5 ml DMSO, and 0.55 mL of carbon disulfide was added at a temperature of 10°C. 0.24 g of sodium hydroxide (powder) was added to the reaction mixture, and the mixture was allowed to stand for 15 minutes. Subsequently, 0.2 mL of MeI was added. The reaction mixture was allowed to stand for another 30 min, and 50 mL of water was added. The resulting white solid was separated by filtration.
Yield: 46%
mp: 108-110°C (EtOH)
MS (m/z): 345 (M⁺)
¹H NMR (DMSO-d₆) : δ 8.69 and 8.41 (two 1H, two s, 2CH), 2.73 (3H, s, CH₃) ppm
Anal. for C₁₁H₅F₆NOS₂:

| | |
|---|---|
| Calc.: | C, 38.26; H, 1.46; N, 4.06; S, 18.57 |
| Found: | C, 38.23; H, 1.50; N, 4.15; S, 18.42 |

### Example 3

### 2-methylthio-6-nitro-4H-1,3-benzothiazin-4-one (compound 3)

Sodium hydroxide (0.4 g; powder) was added to a solution of 1.0 g of 2-chloro-5-nitrobenzamide in 5 ml DMSO at a temperature of 10°C. 0.90 mL of carbon disulfide was added to the solution at a temperature of 10°C, and the reaction mixture was allowed to stand for 15 minutes. MeI (0.31 mL) was added to the reaction mixture at a temperature of 10-20°C. The reaction mixture was allowed to stand for another 30 min, and 50 mL of water was added. The resulting yellow solid was separated by filtration.
Yield: 45%
mp: 168-170°C (ethyl acetate)
MS (m/z): 277 (M⁺)
¹H NMR (DMSO-d₆): δ 8.81(1H, s, CH), 8.43(1H d, CH), 7.91 (1H, d, CH), 2.45 (3H, s, CH₃) ppm
Anal. for C₁₀H₆F₃NOS₂:

| | |
|---|---|
| Calc.: | C, 43.31; H, 2.18; N, 5.05; S, 23.13 |
| Found: | C, 43.46; H, 2.17; N, 5.14; S, 23.19 |

### Example 4

### 8-bromo-2-methylthio-6-trifluoromethyl-4H-1,3-benzothiazin-4-one (compound 4)

Sodium hydroxide (1.5 g; powder) was dissolved in 15 ml DMSO, and 3.36 mL of carbon disulfide was added at a temperature of 10-15°C. 5.6g of 3-bromo-2-chloro-5-fluoromethylbenzamide was added to the solution in small portions at a temperature of 10°C. After 15 minutes, 1.14 mL MeI was added at a temperature of 10-20°C. The reaction mixture was allowed to stand for 30 min, and 130 mL of water was added. The resulting white solid was separated by filtration.
Yield: 41%
mp: 143-145°C(ethanol or hexane)
MS (m/z): 356 (M⁺)
¹H NMR (DMSO-d₆): δ 8.30 and 8.07 (two 1H, two s, 2CH), 2.67 (3H, s, CH₃) ppm
Anal. for C₁₀H₅BrF₃NOS₂:

| | |
|---|---|
| Calc.: | C, 33.72; H, 1.41; N, 4.06; S, 18.57 |
| Found: | C, 33.64; H, 1.55; N, 4.11; S, 18.72 |

### Example 5

### 2-[2S-2-methyl-1,4-dioxa-8-azaspiro[4.5]dec-8-yl]-8-nitro-6-trifluoromethyl-4H-1,3-benzothiazin-4-one (compound 5)

A suspension of 3.0 g of 2-methylthio-8-nitro-6-trifluoromethyl-4*H-*1,3-benzothiazin-4-one (compound 1) in 15 mL of ethanol was treated with 1.5 g of (2*S*)-2-methyl-1,4-dioxa-8-azaspiro[4.5]decane at room temperature. The reaction mixture was heated to 50-60°C for 20 minutes. After cooling, 100 mL of water was added. The resulting light yellow solid was separated by filtration.
Yield: 76% (based on 2-chloro-3-nitro-5-trifluoromethylbenzamide)
mp: 191-193°C (ethyl acetate).
MS (m/z): 431 (M⁺)
¹H NMR (DMSO-d₆): δ 8.81 and 8.76 (two 1H, two s, 2CH), 4.26 (1H, m, CH), 4.11 (1H, m, CH), 3.94 (4H, broad s, 2CH₂) 3,47 (1H, t, CH), 1.80 (4H, broad s, 2CH₂), 1.23 (3H, d, CH₃) ppm
Anal. for C₁₇H₁₆F₃N₃OₛS:

| | |
|---|---|
| Calc.: | C, 47.33; H, 3.74; N, 9.74; S, 7.43 |
| Found: | C, 47.36; H, 3.72; N, 9.84; S, 7.55 |

### Example 6

### 2-(2,2-dimethyl-1,4-dioxa-8-azaspiro[4.5]dec-8-yl)-8-nitro-6-(trifluoromethyl)-4H-1,3-benzothiazin-4-one (compound 6)

Compound 6 was prepared in the same manner as Example 5 but using 2,2-dimethyl-1,4-dioxa-8-azaspiro[4.5]decane as the amine, and a light yellow crystalline solid was obtained.
Yield: 70%
mp: 209-212°C (EtOH)
MS (m/z): 445 (M⁺)
¹H NMR (DMSO-d₆) : δ 8.84 and 8.76 (two 1H, two s, 2CH), 3.93 (4H, broad s, N(CH₂)₂), 3.73 (2H, s, CH₂), 1.79 (4H, broad s, C(CH₂)₂), 1.28 (6H, s, C(CH₃)₂) ppm
Anal. for C₁₈H₁₈F₃N₃O₅S:

| | |
|---|---|
| Calc.: | C, 48.54; H, 4.07; N, 9.43; S, 7.20 |
| Found: | C, 48.56; H, 4.21; N, 9.37; S, 7.30 |

### Example 7

### 2-(4-methylpiperazin-1-yl)-8-nitro-6-(trifluoromethyl)-4H-1,3-benzothiazin-4-one (compound 7)

Compound 7 was prepared in the same manner as Example 5 but using N-methylpiperazine as the amine, and a light yellow crystalline solid was obtained.
Yield: 15%
mp: 180-183°C (EtOH/water)
MS (m/z) : 374 (M⁺)
¹H NMR (DMSO-d₆): δ 8.85 and 8.76 (two 1H, two s, 2CH), 3.90 (4H, broad s, N(CH₂)₂), 2.46 (4H, s, N(CH₂)₂), 2.23 (3H, s, CH₃) ppm
Anal. for C₁₄H₁₃F₃N₄O₃S:

| | |
|---|---|
| Calc.: | C, 44.92; H, 3.50; N, 14.97; S, 8.57 |
| Found: | C, 44.79; H, 3.61; N, 14.42; S, 8.61 |

### Example 8

### 2-[4-(2-methoxyphenyl)piperazin-1-yl]-8-nitro-6-(trifluoromethyl)-4H-1,3-benzothiazin-4-one (compound 8)

Compound 8 was prepared in the same manner as Example 5 but using 1-(2-methoxyphenyl)piperazine as the amine, and a yellow crystalline solid was obtained.
Yield: 89%
mp: 245-247°C (EtOH/DMF or ethyl acetate)
MS m/z 466 (M⁺)
¹H NMR (DMSO-d₆) : δ 8.88 and 8.81 (two 1H, two s, 2CH), 6.87-7.03 (4H, m, C₆H₄), 4.04 (4H, broad s, N(CH₂)₂), 3.13 (4H, broad s, PhN(CH₂)₂) ppm
Anal. for C₂₀H₁₇F₃N₄OS:

| | |
|---|---|
| Calc.: | C, 51.50; H, 3.67; N, 12.01; S, 6.87 |
| Found: | C, 51.43; H, 3.69; N, 11.94; S, 6.98 |

### Example 9

### 8-nitro-2-thiomorpholin-4-yl-6-(trifluoromethyl)-4H-1,3-benzothiazin-4-one (compound 9)

Compound 8 was prepared in the same manner as Example 5 but using thiomorpholine as the amine, and a light yellow crystalline solid was obtained.
Yield: 85%
mp: 202-205°C (EtOH).
MS (m/z): 377 (M⁺)
¹H NMR (DMSO-d₆/CDCl₃); δ 8.84 and 8.75 (two 1H, two s, 2CH), 4.21 (4H, broad s, N(CH₂)₂), 2.82 (4H, broad s, S(CH₂)₂) ppm
Anal. for C₁₃H₁₀F₃N₃O₃S₂:

| | |
|---|---|
| Calc.: | C, 41.38; H, 2.67; N, 11.14; S, 16.99 |
| Found: | C, 41.53; H, 2.59; N, 10.98; S, 17.12 |

### Example 10

### 2-(4-hydroxypiperidin-1-yl)-8-nitro-6-(trifluoromethyl)-4H-1,3-benzothiazin-4-one (compound 10)

Compound 10 was prepared in the same manner as Example 5 but using 4-hydroxypiperidine as the amine, and a light yellow crystalline solid was obtained.
Yield: 93%
mp: 134-136°C (EtOH/water)
MS (m/z): 375 (M⁺)
¹H NMR (DMSO-d₆) : δ 8.85 and 8.75 (two 1H, two s, 2CH), 4.93 (1H, broad s, CH), 4.23 and 3.73 (each 2H, broad s, N(CH₂)₂), 1.86 and 1.53 (each 2H, broad s, C(CH₂)₂) ppm
Anal. for C₁₄H₁₂F₃N₃O₄S:

| | |
|---|---|
| Calc.: | C, 44.80; H, 3.22; N, 11.20; S, 8.54 |
| Found: | C, 44.73; H, 3.27; N, 11.22; S, 8.69 |

### Example 11

### 2-[(2S)-2-methyl-1,4-dioxa-8-azaspiro[4.5]dec-8-yl]-6,8-bis(trifluoromethyl)-4H-1,3-benzothiazin-4-one (compound 11)

A suspension of 0.2 g of 2-methylthio-6,8-ditrifluoromethyl-4*H-*1,3-benzothiazin-4-one (compound 2) in 5 mL of ethanol was treated with 0.08 g of (2*S*)-2-methyl-1,4-dioxa-8-azaspiro[4.5]decane at room temperature. The reaction mixture was heated to 50-60°C for 5 minutes. After cooling, 70 mL of water was added. The resulting white solid was separated by filtration.
Yield: 31%
mp: 189-191°C (EtOH)
MS (m/z): 454 (M⁺)
¹H NMR (DMSO-d₆) : δ 8.68 and 8.36 (two 1H, two s, 2CH), 4.23 (1H, q, CH), 4.09 (1H, t, CH), 3.89 (4H, broad s, 2CH₂), 3,47 (1H, t, CH), 1.80 (4H, broad s, 2CH₂), 1.23 (3H, d, CH₃) ppm
Anal. for C₁₈H₁₆F₃N₂O₃S:

| | |
|---|---|
| Calc.: | C, 47.58; H, 3.55; N, 6.17; S, 7.06 |
| Found: | C, 47.64; H, 3.57; N, 6.13; S, 7.22 |

### Example 12

### 2-(1,4-dioxa-8-azaspiro[4.5]dec-8-yl)-6,8-bis(trifluoromethyl)-4H-1,3-benzothiazin-4-one (compound 12)

Compound 12 was prepared in the same manner as Example 11 but using 1,4-dioxa-8-azaspiro[4.5]decane as amine, and a white crystalline solid was obtained.
Yield: 36%
mp: 195-197°C (EtOH)
MS (m/z): 440 (M⁺)
¹H NMR (DMSO-d₆): δ 8.68 and 8.47 (two 1H, two s, 2CH), 3.98 (4H, s, N(CH₂)₂, 3.89 (4H, broad s, C(CH₂)₂), 1.79 (4H, broad s, C(CH₂)₂) ppm
Anal. for C₁₇H₁₄F₆N₂O₃S:

| | |
|---|---|
| Calc.: | C, 46.37; H, 3.20; N, 6.36; S, 7.28 |
| Found: | C, 46.29; H, 3.25; N, 6.30; S, 7.39 |

### Example 13

### 2-(2-methyl-1,4-dioxa-8-azaspiro[4.5]dec-8-yl)-6-nitro-4H-1,3-benzothiazin-4-one (compound 13)

A suspension of 8.5 g of 2-methylthio-6-nitro-4*H-*1,3-benzothiazin-4-one, (compound 3) in 50 mL of ethanol was treated with 5.25 g of 2-methyl-1,4-dioxa-8-azaspiro[4.5]decane at room temperature. The reaction mixture was heated to 50-60°C for 60 minutes. After cooling, 200 mL of water was added. The resulting white solid was separated by filtration.
Yield: 56%
mp: 216-219°C (EtOH)
MS (m/z): 386 (M⁺)
¹H NMR (DMSO-d₆) : δ 8.82 (1H, s, CH), 8.40 (1H, d, CH), 7.91 (1H, d, CH), 4.27 (1H, m, CH), 4.10 (1H, m, CH), 3.81 (4H, broad s, N(CH₂)₂), 3,47 (1H, t, CH), 1.80 (4H, broad s, C(CH₂)₂), 1.23 (3H, s, CH₃) ppm
Anal. for C₁₇H₁₇F₃N₂O₃S:

| | |
|---|---|
| Calc.: | C, 52.84; H, 4.43; N, 7.25; S, 8.30 |
| Found: | C, 52.88; H, 4.56; N, 7.37; S, 8.17 |

### Example 14

### 8-bromo-2-(2-methyl-1,4-dioxa-8-azaspiro[4.5]dec-8-yl)-6-nitro-4H-1,3-benzothiazin-4-one (compound 14)

A suspension of 5.0 g of 8-bromo-2-methylthio-6-trifluoromethyl-4*H-*1,3-benzothiazin-4-one (compound 4) in 50 mL of ethanol was treated with 2.2 g of 2-methyl-1,4-dioxa-8-azaspiro[4.5]decane at room temperature. The reaction mixture was heated to 50-60°C for 30 minutes. After cooling, 150 mL of water was added. The resulting white solid was separated by filtration.
Yield: 57%
mp: 101-102°C (EtOH)
MS (m/z): 465 (M⁺)
¹H NMR (DMSO-d₆): δ 8.41 (2H, broad s, 2CH), 4.21 (1H, q, CH), 4.09 (1H, t, CH), 3.89 (4H, broad s, 2CH₂), 3,46 (1H, t, CH), 1.80 (4H, broad s, 2CH₂), 1.23 (3H, d, CH₃) ppm
Anal. for C₁₇H₁₆BrNO₃S:

| | |
|---|---|
| Calc.: | C, 43.88; H, 3.47; N, 6.02; S, 6.89 |
| Found: | C, 43.89; H, 3.35; N, 6.12; S, 6.94 |

### Example 15

### 2-[benzyl(methyl)aminol-8-bromo-6-nitro-4H-1,3-benzothiazin-4-one (compound 15)

Compound 15 was prepared in the same manner as Example 14 but using N-methyl-1-phenylmethanamine as the amine, and a white crystalline solid was obtained.
Yield: 47%
mp: 179-181°C (EtOH)
MS (m/z): 429 (M⁺)
¹H NMR (DMSO-d₆) δ 8.45 and 8.41 (two 1H, two s, 2CH), 7.35-7.45 (5H, m, C₆H₅), 5.08 (2H, s, CH₂), 3.33 (3H, s, CH₃) ppm
Anal. for C₁₇H₁₂BrF₃N₂OS:

| | |
|---|---|
| Calc.: | C, 47.57; H, 2.82; N, 6.53; S, 7.47 |
| Found: | C, 47.61; H, 2.81; N, 6.63; S, 7.42 |

### Comparative Example 1 (Method C of WO 2009/010163)

### 2-(2S-2-methyl-1,4-dioxa-8-azaspiro[4.5]dec-8-yl)-8-nitro-6-trifluoromethyl-4-H-1,3-benzothiazin-4-one (compound 5)

4 g of 2-chloro-3-nitro-5-trifluoromethylbenzamide were treated with 3.3 g potassium xanthogenate in 80 mL ethanol at room temperature. The reaction mixture was allowed to stand for about 24 hours, and subsequently 250 mL water was added. The resulting light yellow precipitate was separated by filtration and crystallized from ethanol/water.

1 mmol of the reaction product obtained above was treated with 1.2 mmol of (2S)-2-methyl-1,4-dioxa-8-azaspiro[4.5]decane in 30 mL acetic acid, and the mixture was refluxed for 24 hours. The reaction mixture was evaporated and the residue was treated by 50 ml water, the dark yellow precipitate was separated by filtration, washed with water and twice crystallized from ethanol/water.
Yield: 7% (based on 2-chloro-3-nitro-5-trifluoromethylbenzamide)

## Claims

1. A process which comprises the following step: 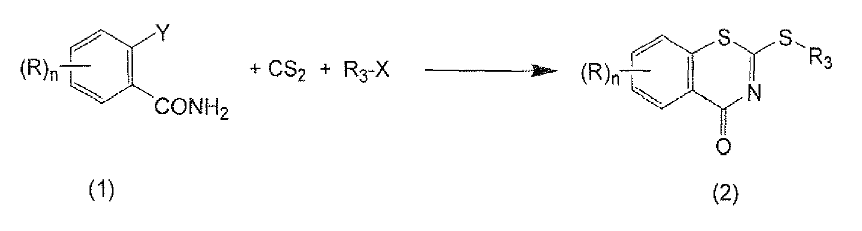 wherein
Y is halogen;
R is independently selected from C₁₋₆-alkyl which may optionally be substituted by halogen, -NO₂, halogen, -CHO, -COOR₄ (wherein R₄ is hydrogen or C₁₋₆-alkyl) and -CN;
R₃ is C₁₋₃-alkyl which may be substituted by halogen; X is halogen; and
n is 0 or an integer of 1 to 4; if n is 2, 3 or 4, multiple Rs may be the same or different.

2. The process of claim 1 which comprises the following step: 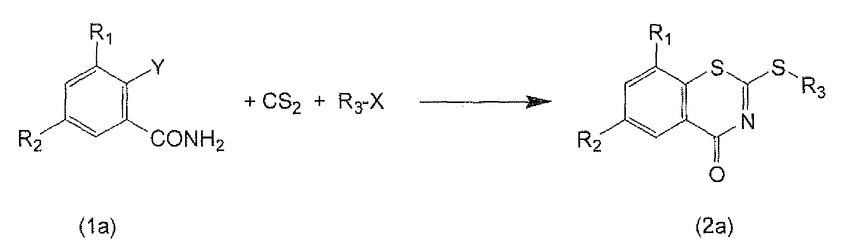 wherein
Y is halogen;
R₁ is H, -CF₃, -NO₂, or halogen;
R₂ is -COOR₄ (wherein R₄ is hydrogen or C₁₋₆-alkyl), -CN, halogen, or -CF₃;
R₃ is C₁₋₃-alkyl which may be substituted by halogen; and
Y is halogen.

3. The process of claim 1 or 2, wherein the reaction is carried out in the presence of a base, preferably in the presence of an alkali metal hydroxide, more preferably in the presence of LiOH, NaOH or KOH.

4. The process according to any one of claims 1-3, wherein R₃-X is selected from CH₃I or CH₂I₂.

5. The process according to any one of claims 1-4 which further comprises the conversion of compound (2) with a primary or secondary amine.

6. The process according to any one of claims 1-4, which further comprises the following step: 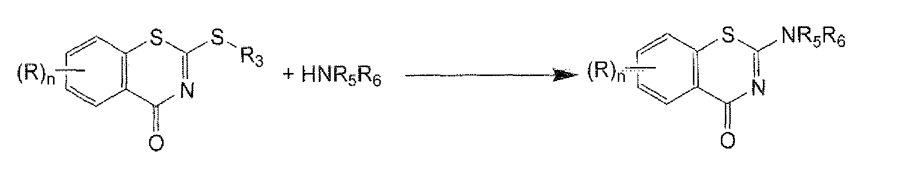 wherein
R, R₃ and n are as defined in claim 1;
R₅ and R₆ are independently selected from H, a saturated or unsaturated, linear, branched or cyclic aliphatic radical having 1-8 carbon atoms, 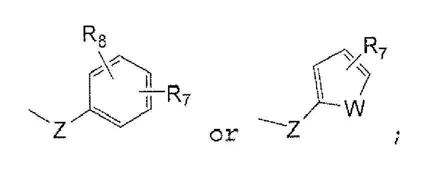 wherein
Z is saturated or unsaturated, linear or branched aliphatic radical having 1-5 carbon atoms;
W is O, NH or N-C₁₋₆-alkyl;
R₇ and R₈ are independently selected from H, C₁₋₆-alkyl, -OH, -OC₁₋₆-alkyl, halogen, or -CN;
R₅ and R₆ may together represent 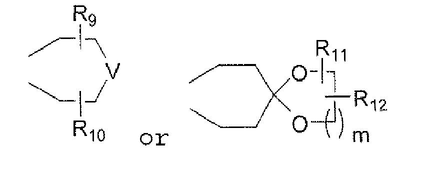 wherein V is O, S, C=O, CHOH, C(R₁₁)₂, NR₁₂;
R₉ and R₁₀ are H, C₁₋₃-alkyl;
R₁₁ is H, a saturated or unsaturated, linear, branched or cyclic aliphatic radical having 1-8 carbon atoms, -OH, -OC₁₋₆-alkyl, phenyl, benzyl, benzoyl, halogen; and multiple R₁₁s may be the same or different;
R₁₂ is H, methyl, benzyl, benzoyl or phenyl (which may be substituted by C₁₋₆-alkyl, -OH, -OC₁₋₆-alkyl, halogen or -CN); and
m is an integer of 1-3.

7. The process according to any one of claims 1-4, which further comprises the following step: 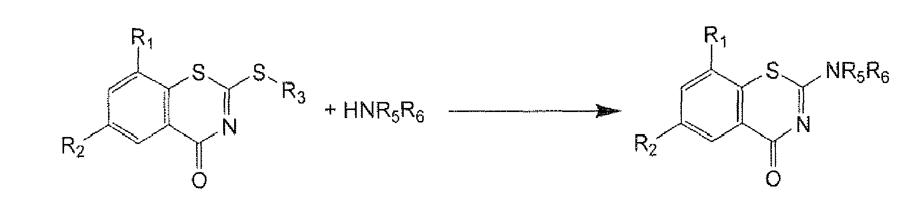 wherein R₁, R₂ and R₃ are as defined in claim 2; and R₅ and R₆ are as defined in claim 6.

8. Use of the process according to any one of claims 1-7 for preparing a 2-aminosubstituted 1,3-benzothiazine-4-one or a pharmaceutical preparation thereof.

9. Use according to claim 8 wherein the 2-aminosubstituted 1,3-benzothiazine-4-one is **characterized by** the following formula: 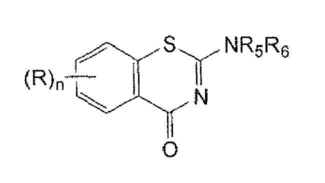 wherein R and n are as defined in claim 1; and R₅ and R₆ are as defined in claim 6

10. Use according to claim 8 or 9, wherein the 2-aminosubstituted 1,3-benzothiazine-4-one is **characterized by** the following formula: 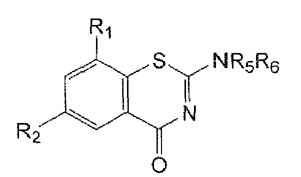 wherein R₁ and R₂ are as defined in claim 2; and R₅ and R₆ are as defined in claim 6.

11. A compound of formula (2): 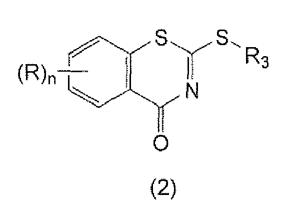 wherein R, n and R₃ are as defined in claim 1.

12. A compound according to claim 11, which is **characterized by** the following formula: 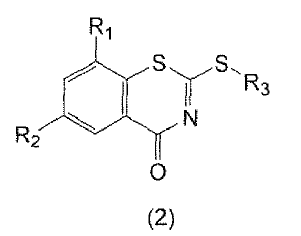 wherein R₁, R₂ and R₃ are as defined in claim 2.

13. A compound according to claim 11 or 12 which is selected from 2-R₃-8-nitro-6-(trifluoromethyl)-4H-1,3-benzothiazin-4-one; 2-methylthio-8-nitro-6-R₂-4*H-*1,3-benzothiazin-4-one; and 2-methylthio-8-bromo-6-R₂-4*H-*1,3-benzothiazin-4-one.

14. A compound according to claim 10 or 11 which is selected from:
2-methylthio-8-nitro-6-(trifluoromethyl)-4H-1,3-benzothiazin-4-one;
2-methylthio-6,8-bis(trifluoromethyl))-4H-1,3-benzothiazin-4-one;
2-methylthio-6-nitro-4H-1,3-benzothiazin-4-one;
8-bromo-2-methylthio-6-trifluoromethyl-4H-1,3-benzothiazin-4-one; and
2-(ethylthio)-8-nitro-6-(trifluoromethyl)-4*H-*1,3-benzothiazin-4-one.

15. Use of the compound according to any one of claims 11-14 for preparing a 2-aminosubstituted 1,3-benzothiazine-4-one or a pharmaceutical preparation thereof.
